# EUROPEAN PATENT APPLICATION

(11) **EP 2 388 329 A2**
(43) Date of publication of application: **23.11.2011**
(21) Application number: 09805852.2
(22) Date of filing: 10.12.2009
(51) Int. Cl.: C12N 15/62, C12N 15/67

(54) **FUSION PROTEINS THE PROCESS TO PREPARATION AND UTILIZATION IN EXPRESSION SYSTEMS OF RECOMBINANT PROTEINS**

(30) Priority: 13.01.2009 PT 09104331
(71) Applicant: Escola Superior Agrária De Coimbra, 3040-316 Coimbra (PT); Hitag - Biotechnology, Lda., 3850-501 Branca (PT)
(72) Inventor: PEREIRA DA CONCEIÇÃO, Maria Antónia, P-3030-330 Coimbra (PT); MARQUES DA COSTA, Sofia Judite, P-4780-301 Santo Tirso (PT); OLIVEIRA CASTRO, António Manuel, P-4405-650 Vila Nova de Gaia (PT); DA SILVA ALMEIDA, André Augusto, P-3850-501 Branca (PT)
(74) Representative: Ferreira, Maria Silvina
(86) International application number: PCT/IB2009/055647
(87) International publication number: WO 2010/082097

(57) **Abstract**

The invention relates to fusion proteins comprising an amino acid sequence corresponding to an H fragment or an amino acid sequence corresponding to a calcium binding protein excreted-secreted by the adult worm of Fasciola hepatica, namely the proteins Fh8 and Fh22, followed by an amino acid sequence corresponding to an unrelated protein fragment or protein, and a process for the preparation thereof and their use in recombinant protein expression systems.

## Description

### Field of the invention

The present invention refers to fusion proteins, a process for the preparation thereof and their use in recombinant protein expression systems.

### Background of the invention

The preparation of specific protein-rich extracts rarely occurs easily from the organisms that produce them. Thus, the production of recombinant proteins is often the only viable way to produce those proteins in required quantities and with the necessary activity levels.

The expression of recombinant proteins is one of the most relevant and most applicable methodologies, both at the research and development levels as well as the industrial level. The possibility to express foreign proteins in well defined models, using bacteria, fungi, or eukaryotic cells, constitutes a tool of great potential and relevance in the field of biotechnology processes, enabling the production of high amounts of the protein of interest.

*E. coli* has been the most widely used system worldwide for proteins not requiring post-translation modifications, such as glycosylation, in order to exhibit activity. The expression of recombinant proteins in *E. coli* is one of the most attractive methods of heterologous production, due to the simplicity of the bacteria, the ability of a rapid and cost-effective growth, the existing physiological knowledge and genetic characterization, and also the availability of advanced genetic tools, among them the large variety of plasmids, fusion recombinant partners and strains with the appropriate mutations. Given the advantages offered by this model, the expression of recombinant proteins in *E. coli* has become one of the most widely used methods for protein production, both at the laboratory and industrial levels.

Several vectors expressing fusion proteins with sequences added for isolation and production stabilization purposes have been described and commercially used. The most widely reported include the production of proteins with the so-called "Histidine-tag", characterized by the addition of a sequence with multiple histidines, the production of fusion proteins with glutathione S-transferase, with maltose binding proteins (MBP), with RNA binding proteins (Document US2004033564), or fusion proteins with ubiquitin (Document WO9701627).

One of the major difficulties when using these expression systems is the production of proteins in the form of inclusion bodies, a common situation that leads to very low yields and the formation of precipitates difficult to handle. This occurrence is particularly common when unstable fragments are used.

The abject of the present invention is based on the development of a methodology that allows increasing significantly the production of recombinant proteins and making their isolation easier, based on the fusion of protein sequences or calcium binding proteins.

The present invention is an alternative to the state of the art systems in the context of the production of recombinant antigens, being described as simple, high yield, and general method that can have significant impact both at the research level and commercial applications.

### Description of the invention

The present invention refers to fusion proteins comprising in their structure: (1) part or the full amino acid sequence of calcium binding proteins excreted/secreted by the adult worm of *Fasciola hepatica;* and an unrelated protein or protein fragment of interest.

It is also an object of the present invention to provide a process for preparing these fusion proteins that allows a very significant increase in the expression and solubilisation of fusion proteins, and consequently of the fragments or proteins added.

The addition of small fragments of calcium binding proteins of the helminth *Fasciola hepatica* - from now on designated H (Helminth) fragments - (SEQ ID NO 2, SEQ ID NO 4), or the same proteins excreted/secreted by the adult worm (SEQ ID NO 1, SEQ ID NO 3), or similar sequences preferably with 90 to 95% of homology, to unrelated protein fragments or proteins allows a significant increase in the production levels of these polypeptides and their solubilization.

Thus, a first aspect of the present invention refers to a fusion protein comprising an amino acid sequence having the structure of the H fragment or of the calcium binding proteins excreted-secreted by the adult worm of *Fasciola hepatica,* followed by an amino acid sequence structurally similar to an unrelated protein fragment or protein. The addition of the fusion tag (H fragment or calcium binding protein) to the unrelated protein can be made at the N or C-terminal position.

Another aspect of the invention refers to an expression vector characterized by comprising a polynucleotide sequence coding for said fusion protein.

Yet another aspect of the invention refers to a host cell comprising the mentioned expression vector.

The present invention also refers to a process for the preparation of fusion proteins, characterized by the fact that a previously mentioned host cell is cultured under conditions appropriate to the expression of said fusion protein, and the fusion protein is isolated.

The invention still refers to the use of said fusion protein for the production of added proteins or protein fragments, in recombinant protein expression systems.

Another aspect of the invention refers to the use of an H fragment or of calcium binding proteins excreted-secreted by the adult worm of *Fasciola hepatica,* for the production of recombinant proteins.

The production of these fusion proteins permits a very significant increase in the expression and solubilazation of added fragments or proteins, thereby enhancing their use for research and development as well as industrial purposes.

### Brief description of figures

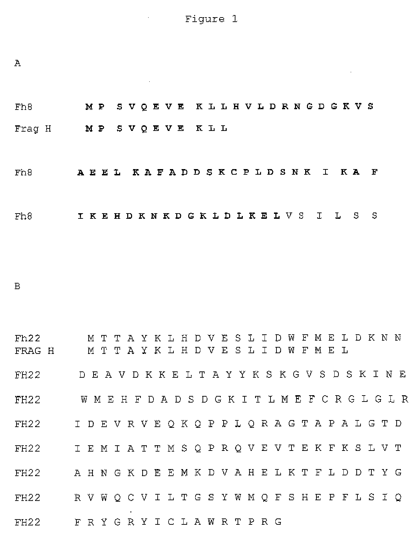

**Figure 1** - Characterization of recombinant proteins and protein fragments, referred to as H fragments, of calcium binding proteins Fh8 and Fh22. A - Amino acid sequence deduced for the polypeptide Fh8 (SEQ ID NO 1); Amino acid sequence deduced for the polypeptide referred to as H fragment of Fh8 (SEQ ID NO 2); B - Amino acid sequence deduced for the polypeptide Fh22 (SEQ ID NO 3); Amino acid sequences deduced for the polypeptide referred to as H fragment of polypeptide Fh22 (SEQ ID NO4).

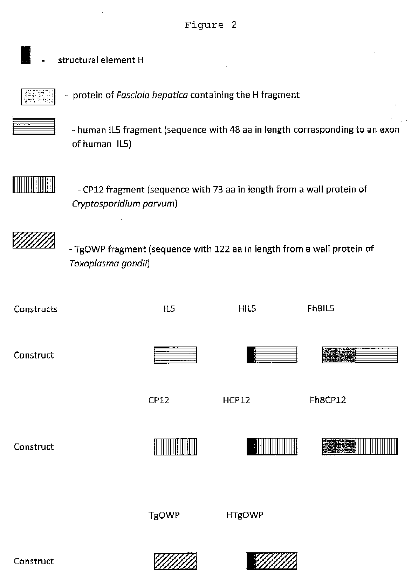

**Figure 2** - Schematic view of subcloning procedures used to evaluate the effect of the H fragment or Fh8 on protein production.

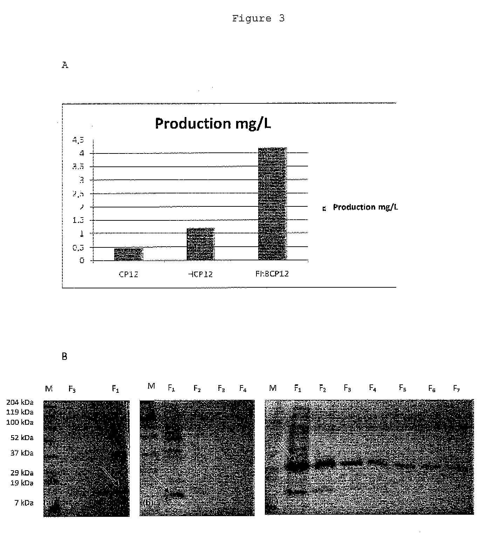

**Figure 3** - Production results for constructs containing the CP12 fragment. A - Graphical representation of protein production; B - Coomassie Blue-stained SDS-PAGE Tris-Tricine gels. M-Prestained SDS-PAGE Standards Marker (Biorad) - (a): F₁, F₂, and F₃ - Fractions 1, 2, and 3 of CP12 collected from the Ni-NTA column. (b): F₁, F₂, F₃, and. F₄ - Fractions 1, 2, 3, and 4 of HCP12 collected from the Ni-NTA column. (c): F₁, F₂, F₃, F₄, F₅, F₆, and F₇ - Fractions 1, 2, 3, 4, 5, 6, and 7 of Fh8CP12 collected from the Ni-NTA column. Arrows indicate the bands of the respective proteins.

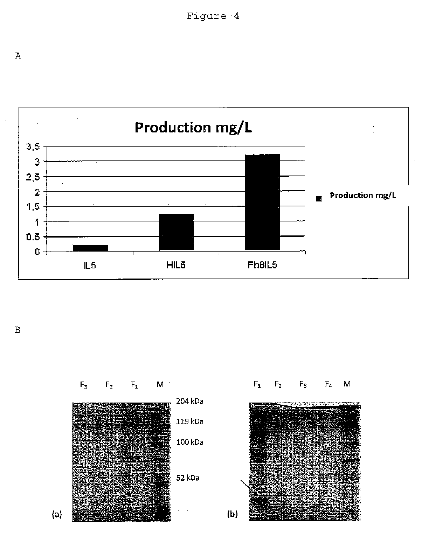

**Figure 4** - Production results for constructs containing the IL5 fragment. A - Graphical representation of protein production; B - Coomassie Blue-stained SDS-PAGE Tris-Tricine gels. M-Prestained SDS-PAGE Standards Marker (Biorad). (a): F₁, F_{2.} and F₃ - Fractions 1, 2, and 3 of IL5 collected from the Ni-NTA column. (b): F₁, F_{2,} F₃, and F₄ - Fractions 1, 2, 3, and 4 of HIL5 collected from the Ni-NTA column. Arrows indicate the bands of the respective proteins.

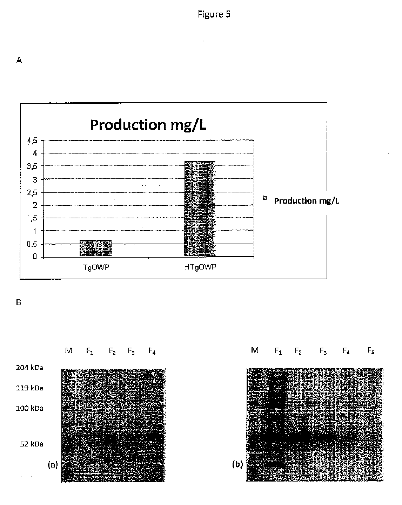

**Figure 5** - Production results for constructs containing the TgOWP fragment. A - Graphical representation of protein production; B - Coomassie Blue-stained SDS-PAGE Tris-Tricine gels. M-Prestained SDS-PAGE Standards Marker (Biorad). (a): F₁, F₂, F₃, and F₄ - Fractions 1, 2, 3, and 4 of TgOWP collected from the Ni-NTA column. (b): F₁, F₂, F₃, F₄, and F₅ - Fractions 1, 2, 3, 4, and 5 of HTgOWP collected from the Ni-NTA column. Arrows indicate the bands of the respective proteins.

### Retailed description of the invention

The present invention relates to recombinant proteins fused to fragments (designated by H fragments (from Helminth) - SEQ ID NO 2 and/or SEQ ID NO 4) Of full amino acid sequences within calcium binding proteins excreted-secreted by the adult worm of *Fasciola hepatica,* namely the protein denoted by Fh8 or fasciolin (Genbank number AF213970), and the family of calcium binding proteins generically known as Fh22, which include the antigens described by EMBL number AJ003822; AJ003821. This strategy allows increasing the production levels of antigens that are fused to the H fragments or H fragment-containing proteins, and also their solubilization, thereby increasing the efficiency of existing recombinant protein production systems. This system allows preventing the production of the antigen of interest in inclusion bodies, thereby making their isolation viable. The biochemical characteristics of calcium binding antigens allow the isolation of the fusion proteins by basic chromatographic techniques, namely ion exchange columns, due to the ability of calcium binding, thereby creating a charged element prone to serve as basis for recovering the fusion antigen.

The presented examples relate to applications using the H fragment of Fh8 (SEQ ID NO 2) or the protein Fh8 (SEQ ID NO 1). Experimental results showed that the H fragment of proteins Fh22 (SEQ ID NO 4) or the proteins Fh22 (SEQ ID NO 3) have a similar action. Likewise, the methodology developed in the context of the present invention is not limited to the production of the fusion proteins shown in the examples, being extensible to any polypeptide without distorting the spirit of the invention.

The several constructs were subcloned into the expression vector pQE (Qiagen) in *Escherichia coli*, resulting in the production of recombinant proteins expressing a sequence of 6 histidines in their N-terminal sequence, such a production being performed in *E. coli* M15 (pREP4) (Qiagen), thereby allowing their isolation by affinity chromatography with a NiNTA agarose column (Qiagen), based on protocols provided by the manufacturer (Castro, 2001, Silva *et al.*, 2004). All production comparisons were performed using this system, the isolation of the recombinant antigen being effected under denaturing conditions to achieve the most effective isolation, in order to compare the production levels of the different constructs.

For the production and isolation of the protein of interest it is possible to use any recombinant protein expression and isolation system. The invention relates to constructs containing the H fragments, or H fragment-containing antigens, in order to solubilize the fusion polypeptides and increase their production. The same methodology may be used in other protein production systems, namely fungi or eukaryotic systems.

One of the principles of the invention is characterized by the addition of H fragments, particularly to the sequence of the N-terminal fragment of Fh8 or Fh22, SEQ ID NO 2 or SEQ ID NO 4, by molecular biology techniques, before the sequence corresponding to the polypeptide to be produced. This construct can be obtained by inclusion of this sequence by molecular biology techniques, namely using appropriate restriction enzymes and adding those restriction sites before and after the fragment sequence, or by other processes, such as the addition of DNA fragments with the sequence of interest (linkers) to a PCR product, or other applicable approaches. The reduced amplitude of the H fragment allows the use of a large variety of strategies for the fusion to the polypeptide of interest.

Another possibility for the construct is the preparation of a fusion protein using the polypeptide corresponding to the sequence of Fh22 or Fh8 and the polypeptide to be produced. The process of inserting the Fh8 or Fh22 sequence can be accomplished using molecular biology techniques, namely using restriction enzymes, such a methodology being used in the demonstration processes.

The invention has been applied to several fragments, namely:
- CP12: CP12 is a surface protein of *Cryptosporidium parvum,* a *coccidium* protozoan belonging to the genus *Cryptosporidium,* phylam *Apicomplexa.* This protein has 104 amino acids (GanSank N° XM_625821), and a molecular weight of 12 kDa, possessing an amino-terminal signal sequence (aa 1-28) and a transmembrane region (aa 12-32), which is a characteristic of surface proteins, and containing neither conserved nor functional domains (Xao *et al.,* 2006). The CP12 fragment used in this work has 213 bp in length and corresponds to the nucleotide sequence of the protein CP12 without the transmembrane domain thereof. The fragment was PCR-amplified and subcloned into the vector pQE (CP12); constructs containing the H fragment followed by CP12 (HCP12), and containing the Fh8 sequence fused to the CP12 sequence (Fh8CP12) were also prepared. The analysis of the production of recombinant antigens isolated under denaturing conditions, using NiNTA agarose resin (QIAGEN), showed an increase of 216% between the production of CP12 and HCP12, and of 954% between the production of CP12 and Fh8CP12.

- IL5: Human interleukin 5 is a hematopoietic growth factor, having a nucleotide sequence of 816 bp coding for 134 amino acids (GenBank N° BC069137.1). The IL5 fragment used for evaluation corresponds to a small part of IL5, consisting of 144 bp corresponding to an exon at the 5' end of IL5 coding for 48 amino acids. The fragment was PCR-amplified and subcloned into the vector pQE (IL5) ; constructs containing the H fragment followed by IL5 (HIL5), and containing the Fh8 sequence fused to the IL5 sequence (Fh8IL5) were also prepared. The analysis of the production of recombinant antigens isolated under denaturing conditions, using NiNTA agarose resin (QIAGEN), showed an increase of 610% between the production of IL5 and HIL5, and of 1600% between the production of IL5 and Fh8IL5.
- TgOWP: TgOWP protein is a oocyst wall protein of *Toxoplasma gondii* with 1846 bp in length coding for 499 amino acids (GenSank N° EU851867.1) . The TgOWP fragment corresponds to the 2^{nd} exon, comprised between the 2875 and 3238 bp. The fragment was PCR-amplified and subcloned into the vector pQE (TgOWP); the construct containing the H fragment-followed by TgOWP (HTgOWP) was also prepared. The analysis of the production of recombinant antigens isolated under denaturing conditions, using NiNTA agarose resin (QIAGEN), showed an increase of 575% between the production of TgOWP and HTgOWP.

In the 3 examples described above, the production levels have increased from levels consistent with their expression in inclusion bodies to production levels corresponding to soluble proteins, and so it can be stated that the placement of H fragments or calcium binding proteins excreted/secreted the adult worm of *Fasciola hepatica* allows increasing very significantly the production of recombinant protein, probably increasing the solubility of the produced antigen.

### Characterization of antigens Fh8 and Fh22 and their H fragments:

The Fh8 and Fh22 antigens were previously isolated and characterized by colleagues belonging to the list of inventors (Castro, 2001, Silva *et al.,* 2004, Eguino *et al.,* 1999), with a brief description of the antigens (Figure 1) being presented in the following.

The isolation of DNA clones (Figure 1) coding for a 69 amino acid polypeptide with a calculated molecular weight of 8 kDa, designated by Fh8 or fasciolin (Genbank number AF213970), was carried out.

The main characteristic of the polypeptide is the existence of two putative calcium binding motifs (EF-hand). The obtained results (Castro, 2001) have demonstrated that Fh8 is an antigen which is present in the excretion/secretion products (ESP) of adult worm of *F. hepatica,* and may appear in the form of polymers.

For characterization purposes and analysis of the activity of Fh8, as well as the potential diagnostic interest, a recombinant antigen, rFh8, containing the sequence corresponding to Fh8, was obtained.

For research purposes, the fragment corresponding to Fh8 was subcloned into the expression vector pQE (Qiagen) in *Escherichia coli.* The production of the recombinant protein rFh8 expressing a sequence of 6 histidines in its N-terminal sequence was performed in *E. coli* M15 (pREP4) (Qiagen), and was isolated by affinity chromatography with a NiNTA agarose column (Qiagen), based on protocols provided by the manufacturer (Castro, 2001, Silva *et al*., 2004) .

Several isolated cDNA clones coded for calcium binding proteins with high homology. These clones were subcloned and analyzed, and some showed identical inserts (EMBL number AJ003822) containing an ORF corresponding to a 191 amino acid peptide with a calculated molecular weight of 22 kDa, which was denoted by Fh22-1. Another clone contained an insert with an ORF for a protein with high homology with the former, which will be characterized later, and was designated Fh22-2 (EMBL number AJ003821).

Fh22 is calcium binding protein with 4 EF-hand structures that has been described as a component of the ESP of adult worms of *F. hepatica* (Eguino *et al.,* 1999). These antigens were subcloned into and produced in the expression vector pQE.

The characterization of Fh8 and Fh22 antigens showed the existence in ESP of a set of proteins with similar structural characteristics. This set of antigens is therefore characterized by the presence of EF-hands, calcium binding structures that regulate the folding of the same, probably causing the stability of both the native protein and the recombinant antigens produced in *E. coli.* Both recombinant proteins are produced at levels higher than 6 mg/L of culture, and both proteins have shown to be produced in soluble form in the cytoplasm of *E. coli,*

The results obtained based on directed mutations at the Fh8 level showed that the production levels observed in *E. coli* depend on structural elements other than those EF-hands, since the levels of recombinant protein mutated at the isolated EF-hands (preventing calcium binding) do not change significantly. These data suggest the existence of other protein structures that ensure such stability. The three-dimensional structure of Fh8 showed that, except for small sequences in the N-terminal (11 aa) and C-terminal (aa 6) ends, all the remaining sequence is involved in or affected by (formation of active centre) the structure resulting from calcium binding to EF-hands. Thus, these studies led to the identification of the N-terminal sequence of Fh8 (later on called the H sequence) as playing a key role in the stability and production of Fh8.

For protein Fh22, the arrangement of HLH (the EF-hand structure, helix-loop-helix - HLH) motifs is similar to Fh8, and as in Fh8, except for the N and C-terminal sequences, the remaining protein is involved in the calcium binding structures, or is affected by them.

The fragments and constructs were subcloned into the expression vector pQE (Qiagen) in *Escherichia coli*. The production of the recombinant protein rFh8 expressing a sequence of 6 histidines in its N-terminal sequence was performed in *E. coli* M15 (pREP4) (Qiagen), and it was isolated by affinity chromatography with a NiNTA agarose column (Qiagen), based on protocols provided by the manufacturer (Castro, 2001, Silva *et al.,* 2004). For the production and isolation of the antigen, any recombinant protein expression and isolation system may be used.

### Strains

In this work, *Escharichia coli XLl Blue* (Stratagene) and *Escherichia coli M15 [pREP4]* (QIAGEN) strains were used for cloning the pGEM-T Easy (Fromega) plasmids and the pQE30 as well as pQE32 (QIAGEN) plasmids, respectively.

*Escherichia coli M15 [pREP4]* strain was used for protein expression.

Plasmid DNA was isolated and purified using the Wizard® Plus SV Minipreps DNA Purification System kit from Promega, from bacterial cultures grown overnight at 37°C, following instructions provided by the manufacturer.

### Constructs

A schematic representation of the constructs prepared to assess the effect of the recombinant antigen Fh8 and its 11 amino acid structural element (H fragment) on increased recombinant protein expression is provided in Figure 2.

All the constructs presented inn Figure 2 were obtained by polymerase chain reaction (PCR), and were cloned into pGEM and then in pQE, as follows.

### PCR

The primers used in the PCRs are described in Table 1.

In order to obtain the H fragment and Fh8RSac fragment, containing BamHI and SacI restriction sites, the pQE30 vector containing the gene coding for the Fh8 polypeptide was used as template for the PCR reaction (Castro, 2001; Silva *et al.,* 2004). The PCR reaction started with a denaturating step at 95 °C for 1 minute, followed by 30 amplification cycles, with denaturation at 94°C for 45 seconds, annealing at 50 °C for 30 seconds and extension at 72 °C for 45 seconds. An additional extension step was performed at 72°C for 11 minutes.

The fragments (CP12, IL5, and TgOWP) chosen to evaluate the ability of the recombinant antigen (and part thereof) to produce a positive effect on protein expression were obtained and amplified by FCR. This PCR reaction also included the addition of the restriction sites of SacI and KpnI restriction enzymes to the respective fragments.

**Table 1. List of prepared constructs, DNA templates, used primers, and respective PCR program**

| **Object** | **DNA sample used as template** | **Identification and sequence of used primers** | **PCR program PCR program** |
|---|---|---|---|
| **H fragment** | Plasmid DNA - pQE30 vector containing the DNA sequence coding for the Fh8 polypeptide | HBamSac : 5`-G A T C C A T G C C T A G T G T T C A A G A G G T T G A A A A A C T C C T T G A G C T C C A T G-3' | 1 min. at 95 °C, followed by 30 amplification cycles (45 s at 94°C, 30 s at 50 °C, and 45 s at 72 °C). 11 min. at 72 °C |
| | | Fh8Rev: 5′-G T T C A C A T A A T A C A C A A T G G T A C C C T A-3′ | |
| **Fh8RSac fragment** | Plasmid DNA - pQE30 vector containing the DNA sequence coding for the Fh8 polypeptide | HFwd: 5'-G G A T C C A T G C C T A G T G T T C A A-3' | 1 min. at 95 °C, followed by 30 amplification cycles (45 s at 94 °C, 30 s at 50 °C, and 45 s at 72 °C). 11 min. at 72 °C |
| | | Fh8RSac: 5'-G T T C A C A T A A T A C A C A A T C T G G A G C T C T G A T G A C A A A A T C-3' | |
| **CP12 fragment** | Genomic DNA of *Cryptosporidium parvum* | CP12Fwd: 5′-C A T A C T G G T A T G A G C T C G A A G G A G T A C - 3′ | 4 min. at 95 °C, followed by 30 amplification cycles (30 s at 95 °C, 30 s at 50 °C, and 1 min. at 72 °C). 7 min. at 72 °C |
| | | CP12Rev: 5'-C A T T A A A A G G T A C C T T T C A T T A T C A A G-3' | |
| **IL5 fragment** | Human genomic DNA | IL5Fwd: 5′-T T C A G A G C C G A G C T C A T G A G G A T G C-3′ | 4 min. at 95 °C, followed by 30 amplification cycles (30 s at 95 °C, 30 s at 50 °C, and 45 s at 72 °c). 7 min. at 72 °C |
| | | IL5Rev: 5'-A A G A A A A T T A C G G T A C C T T A C T C A T T G G C- 3' | |
| **TgOWP fragment** | Genomic DNA of *Toxoplasma gondii* | TgOWP_SacI: 5′-T G T G C C T G T G T G A G C T C C C T C C T G T G-3′ | 4 min. at 95 °C, followed by 30 amplification cycles (30 s at 95 °C, 30 s at 50 °C, and 1 min. at 72 °C) . 7 min. at 72 °C |
| | | TgOWP_KpnI: 5'-T G A T G C G C G G T A C C C T A G G G A A C G A C-3' | |
| **ECP12 fragment** | Binding of PCR product corresponding to SacI-digested H fragment to PCR product corresponding to Sacl-digested CP12 fragment | HFwd: 5'-G G A T C C A T G C C T A G T G T T C A A-3' | 4 min. at 95 °C, followed by 30 amplification cycles (30 s at 95 °C, 30 s at 55 °C, and 45 s at 72 °C) . 7 min. at 72 °C |
| | | CP12Rev: 5'-C A T T A A A A G G T A C C T T T C A T T A T C A A G-3' | |
| **HIL5 fragment** | Binding of PCR product corresponding to SacI-digested H fragment to PCR product corresponding to SacI-digested IL5 fragment | HFwd: 5'-G G A T C C A T G C C T A G T G T T C A A-3' | 4 min. at 95 °C, followed by 30 amplification cycles (30 s at 95 °C, 30 s at 55 °C, and 45 s at 72 °C). 7 min. at 72 °C |
| | | IL5Rev: 5'-A A G A A A A T T A C G G T A C C T T A C T C A T T G G C- 3' | |
| **HTgOWP fragment** | Binding of PCR product corresponding to SacI-digested H fragment to PCR product corresponding to SacI-digested TgOWP fragment | HFwd: 5'-G G A T C C A T G C C T A G T G T T C A A-3' | 4 min. at 95 °C, followed by 30 amplification cycles (30 s at 95 °C, 30 s at 55 °C, and 45 s at 72 °C). 7 min. at 72 °C |
| | | TgOWP_KpnI: 5′-T G A T G C G C G G T A C C C T A G G G A A C G A C-3′ | |
| **Fh8CP12 fragment** | Binding of PCR product corresponding to SacI-digested Fh8RSac fragment to PCR product corresponding to SacI-digested CP12 fragment | HFwd: 5'-G G A T C C A T G C C T A G T G T T C A A-3' | 4 min. at R5 °C, followed by 30 amplification cycles (30 s at 95 °C, 30 s at 55 °C, and 1 min. at 72 °C). 7 min. at 72 °C |
| | | CP12Rev: 5'-C A T T A A A A G G T A C C T T T C A T T A T C A A G-3' | |
| **Fh8IP5 fragment** | Binding of PCR product corresponding to SacI-digested Fh8RSac fragment to PCR product corresponding to SacI-digested IL5 fragment | HFwd: 5'-G G A T C C A T G C C T A G T G T T C A A-3' | 4 min. at 95 °C, followed by 30 amplification cycles (30 s at 95 °C, 30 s at 55 °C and 1 min. at 72 °C) . 7 min. at 72 °C |
| | | IL5Rev: 5′-A A G A A A A T T A C G G T A C C T T A C T C A T T  G G C-3′ | |

The PCR reaction for obtaining CP12 was performed using genomic DNA of *Cxyptosporidium parvum* as template, obtained from oocysts of this parasite, using the DNA extraction kit QIAmp DNA mini kit (QIAGEN), following the protocol provided by the manufacturer.

The PCR reaction started with a denaturating step at 95°C for 4 minutes, followed by 30 amplification cycles consisting of a denaturating step for 30 seconds at 95 °C, an annealing step for 30 seconds at 50 °C, and an extension step for 1 minute at 72 °C. Finally, an additional extension step was performed at 72 °C for 7 minutes.

The PCR reaction for obtaining the IL5 fragment was performed using genomic DNA of *Homo sapiens* as template, obtained from peripheral blood, using the DNA extraction kit QIAmp DNA mini kit (QIAGEN), following the protocol provided by the manufacturer.

The PCR reaction started with a denaturating step at 95 °C for 4 minutes, followed by 30 amplification cycles, consisting of a denaturating step for 30 seconds at 95°C, an annealing step for 30 seconds at 50 °C, and an extension step for 45 seconds at 72 °C. Finally, an additional extension step was performed at 72 °C for 7 minutes.

The PCR reaction for obtaining TgOWP was performed using genomic DNA of *Toxoplasma gondii* as template, obtained from a culture of trophozoytes of this parasite, using the DNA extraction kit QIAmp DNA mini kit (QIAGEN), following the protocol provided by the manufacturer.

The PCR reaction started with a denaturating step at 95 °C for 4 minutes, followed by 30 amplification cycles, consisting of a denaturating step for 30 seconds at 95°C, an annealing step for 30 seconds at 50 °C, and an extension step for 1 minute at 72 °C. Finally, an additional extension step was performed at 72°C for 7 minute.

For the constructs with the H fragment and CP12, IL5 or TgOWP fragments, the binding of the H fragment digested with SacI restriction enzyme to the target fragment digested with the same restriction enzyme is used as template.

For the constructs with the Fh8RSac fragment with CP12 and IL5 fragments, the binding of the Fh8RSac fragment digested with SacI restriction enzyme to the target fragment digested with the same restriction enzyme is used as template.

The PCR program used for obtaining the constructs with the H fragment (namely HCP12, HIL5, and HTgOWP) started with a denaturating step at 95°C for 4 minutes, followed by 30 amplification cycles consisting of a denaturating step at 95 °C for 30 seconds, an annealing step at S5 °C for 30 seconds, and an extension step at 72 °C for 45 seconds. Finally, an additional extension step was carried out at 72 °C for 7 minutes.

The PCR program used for obtaining the constructs with the recombinant antigen Fh8RSac, namely Fh8CP12 and Fh8IL5, started with a denaturating step at 95°C for 4 minutes, followed by 30 amplification cycles consisting of a denaturating step at 95°C for 30 seconds, an annealing step at 55°C for 30 seconds, and an extension step at 72 °C for 1 minute. Finally, an additional extension step was carried out at 72 °C for 7 minutes.

All the PCR reactions were performed with the thermocycler My CyclerTM Thermal Cycler (BioRad).

The PCR reaction mix consisted of 1 µL of sample (DNA template), 2 µL of magnesium chloride, 1 µL of dNTPs (Roche), 1 µL of forward primer and 1 µL of reverse primer, 5 µL of Taq polymerase enzyme buffer (Thermo Scientific), I unit/µL of Taq polymerase enzyme (Thermo Scientific), and distilled water to a final volume of 50 µL.

### DNA extraction and purification from agarose gals

PCR products or DNA bands resulting from digestions with restriction enzymes were isolated in agarose gel using the illustxaTM GFX PCR DNA & Gel Band Purification kit (GE Healthcare), following the procedure now described.

### pGEM Vector binding

The binding reaction to pGEM-T Easy vector was carried out using a mixture consisting of 3 µL of the DNA sample (PCR product or restriction enzyme-digested product), 1 µL of pGEM-T Easy vector (Promega), 5 µL of DNA ligase enzyme buffer 2X (Promega), and 1 µL of DNA T4 ligase enzyme (Promega), to a final volume of 10 µL. This reaction was conducted at room temperature overnight or at 37 ° C for 1 hour and 30 minutes.

### Confirmation of transformants by restriction enzyme digestion

After the binding reaction to pGEM vector, *E. coli XL1 Blue* was transformed with the binding product. The cells were then plated onto LB/Ampicillin/X-Gal/IPTG plates and incubated overnight at 37 °C. The white transformants were transferred to liquid cultures of LB/Ampicillin, and then the plasmid DNA extraction protocol was carried out from *E. coli.*

Confirmation of transformants was done by digestion with EcoRI restriction enzyme (Promega), using 7 µL of selected clones, 2 µL of H buffer 10X and 1 µL of EcoRI, to a final volume of 10 µL. The reaction was conducted for 2 to 3 hours at 37 °C, and the result of the digestion was observed on an agarose gel with an appropriated percentage (w/v).

### pQE Vector binding

The inserts resulting from the digestions, performed in this work, with restriction enzymes were inserted into the pQE vector using a mixture consisting of 6 µL of insert, 2 µL of pQE vector, 1 µL of ligase buffer 10X (Promega), and 1 µL of NOVA T4 Ligase enzyme (Promega) . This reaction was conducted at room temperature overnight or at 37 °C for 1 hour and 30 minutes.

After binding of the insert to the pQE vector, *E. coli M15 [pREP4]* was transformed with the binding product. The cells were then plated onto LB/Ampicillin/Kanamycin plates and incubated overnight at 37 °C. The obtained transformants were transferred to liquid cultures of LB/Ampicillin/Kanamycin, and then the plasmid DNA extraction protocol was carried out from *E. coli.*

Confirmation of transformants was done by digestion with KpnI and BamHI restriction enzymes (Promega), both in the case of negative controls of the protein expression evaluation, pQECP12, pQEIL5, and pQETgOWP, and in the case of constructs with the Fh8 recombinant antigen or with the H fragment.

The first digestion was performed with KpnI, using a mixture consisting of 26 µL of DNA, 3 µL of J buffer (Promega), and 1 µL of KpnI (Promega), to a final volume of 30 µL. 10 µL of this digestion were analyzed on agarose gel, and the second digestion of the resulting 20 µL was performed with DamHI, using a mixture consisting of 2 µL of K buffer 10X (Promega) and 1 µL of BanHI (Promega). The result of the digestion was observed on agarose gel with an appropriated percentage (w/v).

### Construct sequencing

All the constructs prepared in this work, with the inserts in the pGEM and pQE vectors, were confirmed by sequencing at Eurofins MWG Operon (Germany).

### Expression analysis of constructs with the recombinant antigen/H fragment, unbar denaturating conditions

A 200 mL pre-culture was prepared, being grown overnight at 37°C with stirring, and 2 litres of culture were induced by mixing 100 mL of the saturated culture and 900 mL of LB medium containing 100 µg/mL Ampicillin, 50 µg/mL Kanamycin and 1 mM IPTG. After 5 hours of incubation, cells were collected by centrifugation at 4000 rpm, 4 °C for 20 minutes. Cell lysis was carried out by incubating cells with 40 mL of 8 M urea buffer, pH 8.0, overnight with stirring. The extract was centrifuged at 13000 rpm, at room temperature for 15 minutes, and the supernatant was collected. After recovering the supernatant, the same was filtered in a glass wool column before being applied to the Ni-NTA column (Amersham Biosciences), pre-equilibrated with 8 M urea pH 8.0.

The supernatant flowed through the column by means of gravity, and the column was then washed with 5 CV (column volumes) of 8 M urea buffer pH 8.0, followed by 5 CV of 8 M urea buffer with 10% glycerol, pH 6.5. Elution occurred with 8 M urea buffer, pH 4.5, and 4 mL fractions were collected.

Eluted fractions were quantified by Bradford assay and analyzed on SDS-PAGE Tris-Tricine gel, as described in the following.

### Protein quantification

Protein quantification was performed by Bradford assay with the Protein Assay (Biorad) reagent diluted 1:5, and the optical density was read at a wavelength of 595 nm. The calibration curve was obtained from optical density readings, at 595 nm, of solutions of defined bovine sera albumin (BSA) concentrations with this reagent.

### Polyacrylamide gel electrophoresis SDS--PAGE Tris-Tricine

Tris-Tricine gels used to analyze the collected fractions were based on the Tris-tricine systems of Schagger, H. and Jagow, G. (1987) and on the SDS-PAGE system of Laemmli (1970). The adopted system consisted of two gels: a 15% resolving gel and a 4% stacking gel. The resolving gel contained 3.3 mL of 30% acrylamide, 2.205 mL of gel buffer, 705 µL of glycerol, 367.5 µL of water, 150 µL of 10% APS4, and 9 µL of TEMED. The stacking gel contained 700 µL of 30% acrylamide, 1.25 mL of gel buffer, 3 mL of water, 200 µL of 10% APS, and 5 µL of TEMED.

The electrophoresis system used comprised two, upper (next to gels) and bottom, reservoirs with cathode buffer and anode buffer, respectively. A 100 V difference of potential (dop) was applied to the stacking gel and a 150 V dop was applied to the resolving gel.

Before being applied onto the gel, the samples (under native or denaturating conditions) were treated with sample Tris-Tricine buffer 1X. The samples under native conditions were also placed in the bath at 100 °C for 2 minutes, then staying at 4 °C until being loaded onto the gel.

Gels were stained with Coomassie blue and unstained with a decolouring solution.

### Examples

The recombinant antigen studied in this work is characterized by a good expression yield, and can be easily isolated by ion exchange chromatography.

The strategies presented in the following were based on these premises, and three fragments (IL5, CP12, and TgOWP) were chosen to evaluate the increase in expression yields resulting from the addition of the H fragment or H fragment-containing protein.

### Example 1 - HCP12 and HIL5 constructs

In order to obtain these constructs we first obtained the H fragment. A PCR was carried out using primers H and Fh8Rev (see Table I). Primer H allows inserting a SacI restriction site after the codon of the 11^{th} amino acid of Fh8. That PCR product was isolated and then digested with SacI restriction enzyme. The SacI-digested N-terminal fragment (H fragment) was then isolated.

Secondly, IL5 (primers IL5Sac and IL5Kpn) and CP12 (primers CP12Sac and CP12Rpn) fragments were amplified using isolated human genomic DNA (gDNA) and gDNA of *Czyptosporadaum parvum,* respectively, as templates. After the isolation of the PCR products, digestion with SacI and respective isolation of Sac-digested fragments were performed.

The Sac-digested H fragment was bound to the IL5 and CP12 fragments digested with SacI. The HIL5 fragment was obtained by PCR using that binding as template and Primer H and primer IL5Kpn as primers. The HCP12 fragment was obtained in a similar way, using Primer H and primer CP12 Kpn. Both of these fragments were subcloned into the pQE30 vector using BamHI and KpnI restriction enzymes.

### Example 2 - HTgOWP construct

In order to obtain the HTgOWP fragment, pQEHCP12 vector was digested with KpnI and SacI, thereby removing the CP12 fragment from the vector, and thus obtaining pQE30 H.

The TgOWP fragment was produced by PCR using gDNA of *Toxoplasma gondii* as template, and primers TgOWPSac and TgOWPKpn.

The PCR reaction allowed the addition of SacI and KpnI enzyme restriction sites to the DNA template sequence of TgOWP. This product was then purified onto agarose gel and bound to the pGEM vector. Subsequently, the digestion with KpnI and SacI restriction enzymes was performed, followed by isolation of the TgOWP fragment digested with SacI and KpnI. This fragment was bound to the pQE30H vector digested with SacI and KpnI, thereby obtaining the construct pQEHTgOWP.

### Example 3 pQECP12, pQETT.5, and pQETgOWP constructs

Vectors containing the H fragment (pQEHCP12, pQEHIL5, and pQETgOWP) were used for preparing the respective negative controls. As such, HCP12, HIL5, and HTgOWP fragments were used as starting point for obtaining the negative controls.

Since the procedure was similar for the three vectors, the example for preparing the pQECP12 recombinant vector is presented in the following. The pQEHCP12 vector was digested with SacI enzyme and subsequently purified on agarose gel. After the purification step, the pQEHCP12 vector was also digested with EcoRI restriction enzyme, present in the pQE30 vector sequence. This digestion allowed the removal of the H fragment as well as a small part of the pQE30 vector. pQEHCP12, pQEHIL5, and pQEHTgOWP vectors, digested with SacI and EcoRI restriction enzymes, were purified on agarose gel, and then received a digestion fragment of the pQE30 empty vector. For that purpose it was necessary to digest this vector with the same restriction enzymes, SacI and EcoRI. The lowest molecular weight band obtained, corresponding to the small part of pQE30 vector between the EcoRI and SacI restriction sites (about 70 bp), was purified on agarose gel and bound to pQE30 with the CP12 fragment previously obtained, resulting in pQECP12 vector. The pQECP12 vector was then transformed in *E. coli M15 [pREP4].*

### Example 4 - pQEFh8CP12 and pQEFh8IL5 constructs:

### Fh8RSac fragment

The Fh8RSac fragment was obtained by PCR modifying an Fh8 template sequence and using the primers Fh8For and Fh8RSac, wherein the SacI enzyme restriction site was added before the Fh8 stop codon. The PCR reaction product was then purified on agarose gel and bound to pGEM vector.

The PCR product obtained was then digested with SacI restriction enzyme, resulting in a SacI enzyme-digested Fh8RSac fragment. This fragment was then purified using an agarose gel and was bound to CP12 and IL5 fragments, digested with SacI enzyme.

### Fh8CP12 and Fh8IL5 fragments

As mentioned before, CP12 as well as IL5 fragments were obtained using a similar strategy, from a PCR reaction to which SacI and KpnI restriction sites were added. After IL5 or CP12 amplification, these fragments were digested with SacI restriction enzyme and bound to Fh8RSac fragment, also previously digested with SacI. Fh8CP12 fragment was then obtained using Fh8CP12 binding as template and primers Fh8For and CP12Kpn. FhSIL5 fragment was obtained in a similar way, using Ph8IL5 binding as template and primers Fh8For and IL5Kpn. The PCR fragments were isolated, and subcloned firstly into pGEM vector and Later into pQE vector.

### Recombinant antigen expression analysis by Ni-NTA column, under denaturating conditions.

IL5, HIL5, Fh8IL5, CP12, HCP12, Fh2CP12, TgOWP, and HTgOWP proteins were expressed in *E. coli M15 [pREP4],* being evaluated in 2 L cultures with LB medium and respective antibiotics. IL5, CP12, and TgOWP proteins were used as negative control of protein expression analysis of the respective fusion proteins. All cultures were induced with 1 mM IPTG at 37 °C for 5 hours, cell extracts were prepared and the soluble fractions analysed under denaturating conditions (8 M Urea) by Ni-NTA columns (Amersham Biosciences).

The expression of the fusion proteins, as well as respective negative controls was quantified by Bradford assay and evaluated on SDS-PAGE Tris-Tricine gels.

### Protein expression quantification

Protein expression quantification was made by Bradford assay, and Figures 3, 4, and 5 show the protein expression levels between the fusion proteins, HIL5 and HCPI2 - Fh8CP12 and FH8IL5, and respective negative controls, IL5 and CP12.

HIL5 expression (1.22 mg/L) was approximately 6 fold higher than the respective negative control, IL5 (0.2 mg/L). This increase became even more significant when IL5 was fused to Fh8, the expression (3.2 mg/L) being approximately 16 fold higher than the respective negative control, IL5.

CP12 fusion to recombinant antigen H fragment (1.21 mg/L) resulted in a 3 fold increase in protein expression when compared to the respective negative control, CP12 (0.44 mg/L). This increase was even more significant for CP12 fused to Fh8, the expression (3.94 mg/L) being approximately 9 fold higher than the respective negative control, CP12- Fh8CP12 fusion protein showed a protein expression 3 fold higher than HCP12 fusion protein.

Negative control proteins, IL5 and CP12, are proteins that under normal conditions (without addition of part or the full recombinant antigen) are not produced in soluble form in *E. coli,* possibly being directed to inclusion bodies during the refolding process (Proudfoot *et al.*, 1990; Yao *et al.,* 2006).

Furthermore, the sequences used in this work correspond to a small part of IL5 and CP12 proteins, adding to the difficulty of their stabilization during refolding. The expression yields obtained for these proteins are below the solubility limit (usually between 0.5 and 1 mg/L), highlighting their difficult soluble expression.

The addition of H fragment or the full recombinant antigen stabilizes IL5 and CP12 fragments, causing an increase in their expression. More impressive than this expression increase are the changes occurring at the cellular level, causing a shift from an insoluble structure to a soluble different one.

The expression of HTgOWP fusion protein (3.68 mg/L) was approximately 6 fold higher than the negative control, TgOWP (0.64 mg/L). The expression of this protein is in the solubility threshold. What should be noticed, however, is the significant increase in expression resulting from the addition of the H fragment. This fragment, as with IL5 and CP12, will stabilize the protein, leading to its soluble production. Since TgOWP production with H fragment added reached high expression levels, the need to add TgOWP with the recombinant antigen sequence (Fh8) is not justified.

Protein expression with the recombinant antigen was also evaluated by electrophoresis (Figures 3, 4, and 5) under denaturating conditions (SDS-PAGE Tris-Tricine).

### Bibliography

Castro, A. M. (2001). Preparation and characterization of recombinant proteins homologous antigen excreted / secreted by adult worms of Fasciola hepatica. PhD Thesis. University of Oporto.
Eguino, A. R., Marchini, A., Young, R., Castro, A., Boga, J., Martin-Alonso, J. and Parra, F. (1999). Cloning and expression in Escherichia coli of the gene encoding the calcium-binding protein. Molecular and Biochemical Parasitology. 101: 13-21.
Laemmli, U.K., (1970). Cleavage of structural proteins during the assembly of the head of bacteriophage T4. Nature, 227: 680-685.
Proudfoot, A., Fattah, D., Kawashima, E., Bernard, A. and Wingfield, P. (1990). Preparation and characterization of human interleukin-5 expressed in recombinant Escherichia coli. Biochemical Journal. 270: 357-361.
Schägger, H. and Jagow, G. (1987). Tricine-sodium dodecyl sulfate-Polyacrilamide gel electrophoresis for the separation of proteins in the range from I to 100 kDa. Analytical Biochemistry, 166: 368-379.
Seong, B. L., Choi, S., and Shin H. C. 2004. Method for increasing solubility of target protein using RNA-binding protein as fusion partner. U.S. Patent 2004033564. 2004-02-19.
Silva, E., Castro, A., Lopes, A., Rodrigues, A., Dias, C., Conceiçào, A., Alonso, J., Correia da Costa, J. M., Bastos, M., Parra, F., Addresses-Ferreira, P., and Silva, M. (2004). The recombinant antigen recognized by Fasciola hepatica-infected hosts. The Journal of Parasitology. 90 (4), 746-751.
Yao, L., Yin, J., Zhang, X., Liu, Q., Li, J., Chen, L., Zhao, Y., Gong, P. and Liu, C. (2006). Cryptosporidium parvum: Identification of a new surface adhesion protein on sporozoite and oocyst by screening of a phage-display cDNA library. Experimental Parasitology. Doi: 10.1016/j.exppara.2006.09.018.
Yost, P. B., Pilon, A. L., Lohne, G. L., and S. Roberts F. 1997. High-level expression and efficient recovery of ubiquitin fusion proteins from Escherichia coli. WO9701627 1997-01-16.

## Claims

1. A fusion protein **characterized by** the fact that one comprises the amino acid sequence identical or 90 to 95% similar to H fragment or protein binding to calcium excreted-secreted by adult worms of Fasciola hepatica, followed by an amino acid sequence corresponding to a protein or protein fragment not related with the fusion protein, being the addition of the fusion tags to the not related protein made at position N or C-terminal.

2. Fusion protein according to claim 1, **characterized by** the fact that the H fragment corresponding to a fragment structurally similar to N-terminal 11 amino acids of the protein FH8, SEQ ID NO 2.

3. Fusion protein according to claim 1, **characterized in that** the amino acid sequence to a protein binding to calcium is structurally similar to excreted-secreted by adult worms of Fasciola hepatica be FH8, SEQ ID NO 1.

4. Fusion protein according to claim 1, **characterized in that** the amino acid sequence corresponding to a unrelated protein fragment or protein to be the CP12.

5. Fusion protein according to claim 1, **characterized in that** the amino acid sequence corresponding to a unrelated protein fragment or protein to be the IL5.

6. Fusion protein according to claim 1, **characterized in that** the amino acid sequence corresponding to a unrelated protein fragment or protein to be the TgOWP.

7. An expression vector **characterized by** the fact that it comprise a polynucleotide sequence that encodes the fusion protein according to any one of claims 1 to 6.

8. A host cell **characterized by** the fact that to comprise the expression vector according to claim 7.

9. A process for the preparation of fusion proteins according to any one of claims 1 to 6, **characterized by** the fact that cultivating a host cell according to claim 8 under conditions appropriate for expression of the fusion protein and isolating the fused protein.

10. Use of the fusion protein according to any one of claims I to 7 for the production of proteins or protein fragments added in expression systems.

11. Use of a fragment H or calcium binding proteins, excreted-secreted by adult worms of Fasciola hepatica in the production of recombinant proteins.


SEQUENCE LISTING

<110> Escola Superior Agrária de Coimbra

<120> FUSION PROTEINS, PREPARATION PROCESS THEREOF AND THEIR USE IN RECOMBINANT PROTEIN EXPRESSION SYSTEMS

<130> PAT 39544-08

<160> 4

<170> PatentIn version 3.3

<210> SEQ ID NO 1

<211> 69

<212> PRT

<213> Fasciola hepatica

<400> 1

Met Pro Ser Val Gln Glu Val Glu Lys Leu Leu His Val Leu Asp Arg

1               5                   10                  15

Asn Gly Asp Gly Lys Val Ser Ala Glu Glu Leu Lys Ala Phe Ala Asp

            20                  25                  30

Asp Ser Lys Cys Pro Leu Asp Ser Asn Lys Ile Lys Ala Phe Ile Lys

        35                  40                  45

Glu His Asp Lys Asn Lys Asp Gly Lys Leu Asp Leu Lys Glu Leu Val

    50                  55                  60

Ser Ile Leu Ser Ser

65

<210> SEQ ID NO 2

<211> 11

<212> PRT

<213> Fasciola hepatica

<400> 2

Met Pro Ser Val Gln Glu Val Glu Lys Leu Leu
1               5                   10

<210> SEQ ID NO 3

<211> 190

<212> PRT

<213> Fasciola hepatica

<400> 3

Met Thr Thr Ala Tyr Lys Leu His Asp Val Glu Ser Leu Ile Asp Trp
1               5                   10                  15
Phe Met Glu Leu Asp Lys Asn Asn Asp Glu Ala Val Asp Lys Lys Glu
            20                  25                  30
Leu Thr Ala Tyr Tyr Lys Ser Lys Gly Val Ser Asp Ser Lys Ile Asn
        35                  40                  45
Glu Trp Met Glu His Phe Asp Ala Asp Ser Asp Gly Lys Ile Thr Leu
    50                  55                  60
Met Glu Phe Cys Arg Gly Leu Gly Leu Arg Ile Asp Glu Val Arg Val
65                  70                  75                  80

Glu Gln Lys Gln Pro Pro Leu Gln Arg Ala Gly Thr Ala Pro Ala Leu
                85                  90                  95

Gly Thr Asp Ile Glu Met Ile Ala Thr Thr Met Ser Gln Pro Arg Gln
            100                 105                 110
Val Glu Val Thr Glu Lys Phe Lys Ser Leu Val Thr Ala His Asn Gly
        115                 120                 125
Lys Asp Glu Glu Met Lys Asp Val Ala His Glu Leu Lys Thr Phe Leu
    130                 135                 140
Asp Asp Thr Tyr Gly Arg Val Trp Gln Cys Val Ile Leu Thr Gly Ser
145                 150                 155                 160
Tyr Trp Met Gln Phe Ser His Glu Pro Phe Leu Ser Ile Gln Phe Arg
                165                 170                 175
Tyr Gly Arg Tyr Ile Cys Leu Ala Trp Arg Thr Pro Arg Gly
            180                 185                 190

<210> SEQ ID NO 4
<211> 20
<212> PRT
<213> Fasciola hepatica

<400> 4

Met Thr Thr Ala Tyr Lys Leu His Asp Val Glu Ser Leu Ile Asp Trp
1               5                   10                  15
Phe Met Glu Leu
            20